# EUROPEAN PATENT SPECIFICATION

(11) **EP 3 558 469 B1**
(45) Date of publication and mention of the grant of the patent: **20.05.2020**
(21) Application number: 17826454.5
(22) Date of filing: 12.12.2017
(51) Int. Cl.: A61Q 19/08, A61Q 19/00, A61K 8/92, A61K 8/368, A61K 8/34, A61K 8/36, A61K 8/46, A61K 8/55, A23L 3/34

(54) **MALODOR REDUCTION OF COSMETIC COMPOSITIONS**
GERUCHSREDUZIERUNG FÜR KOSMETIKZUSAMMENSETZUNGEN
RÉDUCTION DES MAUVAISES ODEURS DE COMPOSITIONS COSMÉTIQUES

(30) Priority: 22.12.2016 EP 16206340
(43) Date of publication of application: 30.10.2019
(73) Proprietor: Unilever PLC, London, Greater London EC4Y 0DY (GB); Unilever N.V., 3013 AL Rotterdam (NL)
(72) Inventor: TOMASHEVSKAIA, Marina, Trumbull, Connecticut 06611 (US); PEHRATOVIC, Hasiba, Trumbull, Connecticut 06611 (US)
(74) Representative: James, Helen Sarah
(86) International application number: PCT/EP2017/082400
(87) International publication number: WO 2018/114478

(56) References cited:
- WO-A1-01/08653
- WO-A1-2005/079740
- WO-A1-2016/000863
- DE-A1- 4 320 871
- FR-A1- 2 968 955
- US-A- 5 472 705
- US-A1- 2003 073 771
- US-A1- 2003 185 865
- US-A1- 2008 219 938
- KANEBO: "Skin moisturiser - has hydroxy ethane diphosphite, diethylenetriamine-5- acetate or L-ascorbic acid-2-phosphate, used f preventing skin chloasma or freckles", DERWENT, 15 November 1996 (1996-11-15), - 1 January 1998 (1998-01-01), XP002197098,
- DREHER M L ET AL: "Hass avocado composition and potential health effects", CRITICAL REVIEWS IN FOOD SCIENCE AND NUTRITION, TAYLOR & FRANCIS, USA, vol. 53, no. 7, 1 May 2013 (2013-05-01), pages 738-750, XP002734320, ISSN: 1040-8398, DOI: 10.1080/10408398.2011.556759 [retrieved on 2013-05-02]

## Description

### Field of the invention

The present invention is directed to a skin care composition, a method for stabilizing a component, like an active, and reducing malodor in a skin care composition. More particularly, the invention is directed to a skin care composition comprising radical scavenger, a peroxide decomposer or both that are suitable to prevent formation of compounds that have been proven to yield offensively unpleasant odors in compositions, like lotions, creams, shampoos and body washes. Such a scavenger and decomposer (or mixture of the same) unexpectedly stabilize polyunsaturated fatty acids, esters and/or salts thereof in the skin care compositions by preventing oxidation of the same and thereby reducing the formation of components that generate odors that are unacceptable to consumers. Moreover, such a scavenger and decomposer surprisingly do not negatively impact the desired sensory attributes resulting from the use of a skin care composition comprising the same and they allow for composition packaging in open and non-airless packages.

### Background of the invention

A wide variety of skin care compositions tend to generate malodors after coming into contact with air, bacteria, skin or combinations of the same for prolonged periods of time. In fact, many skin care compositions comprise actives that, for example, oxidize, thereby generating volatile components that result in malodor.

Attempts at reducing malodor in skin care compositions have been made. For example, fragrances have been used in skin care compositions to mask malodors. Use of fragrances, however, is not always desirable since many consumers wish to use skin care compositions that are free of fragrances, due to various skin sensitivities and allergies. Also, fragrances within a product tend to have a shorter life than the product itself. Therefore, malodor masking may not be achieved during an entire product life. Other attempts at reducing malodor in skin care compositions include using reduced amounts of components prone to oxidation and thus proven to be unstable in end use formulations. This approach is not advantageous because less component, often an active, is delivered to the consumer.

There is increasing interest to develop skin care compositions that have excellent sensory characteristics, are stable and free of malodor, and especially, skin care compositions that are free of malodor resulting from active decomposition, active that consumers are expecting to be present to yield a noticeable benefit when the skin care composition is applied. This invention, therefore, is directed to a skin care composition comprising radical scavenger and/or peroxide decomposer. The skin care composition made according to this invention is surprisingly free of malodor originating from components that, for example, are an *in situ* product of active oxidation and where such active is expected to deliver benefit to the consumer.

### Additional Information

Efforts have been disclosed for making cosmetic compositions. In World Application No. WO 93/18130, malodor personal cleansing bars with zeolite are described.

Other efforts have been disclosed for making cosmetic compositions. In U.S. Application No. 2006/0135385 A1, toilet bar compositions with pyran odor masking agents are described.

Still other efforts have been disclosed for making consumer product compositions with reduced odor. In European Patent Application No. EP 0063899 A2, fabric conditioning compositions whereby the same exhibit excellent deodorizing effects against a wide range of malodor ingredients.

Even other efforts have been disclosed for making cosmetic compositions. In Japanese Application No. JP 2004290573 A, deodorants having elasticity and flexibility are described whereby the same uses clay as a swelling agent. WO2016/000863 describes nitrogen containing borane stabilized skin care compositions. WO2005/079740 describes stabilization of personal care products against color degradation with substituted ureas. None of the additional information above describes a skin care composition that has a radical scavenger, a peroxide decomposer or both that reduce malodor formation by preventing *in situ* breakdown of, for example, active expected to deliver benefit to the consumer.

### Summary of the Invention

The present invention is directed to a topical composition comprising:
(a) a component that can produce *in situ* a product of oxidation, the component comprising polyunsaturated fatty acid, ester or salt thereof, the component further comprising conjugated linoleic acid, low oleic sunflower seed oil or both;
(b) 0.1 to 1 weight percent radical scavenger comprising pentaerythritol tetrakis (3-(3,5-di-tert-butyl-4-hydroxyphenol)propionate) and 0.1 to 1 weight percent, peroxide decomposer comprising didodecyl 3,3' thiodipropionate; and
(c) oil carrier comprising caprylic/capric triglyceride.

The present invention is also directed to emulsions comprising the compositions of the invention.

In a further aspect, the invention is directed to cosmetic methods of improving skin characteristics by contacting the skin with the compositions of this invention.

Additional aspects of the present invention will more readily become apparent from the description and examples which follow.

Skin, as used herein, is meant to include skin on the face, neck, chest, back, arms (including underarms), hands, legs, buttocks and scalp. Hair includes hair on the head, and nails include both nails on the feet and hands. Active, as used herein, is meant to include a component that improves a body characteristic after topical application like a skin, hair and/or nail characteristic and/or benefits the same wherein the active can be, and preferably is, an active in a cream, pump or aerosol spray, serum, lotion, balm, deodorant, or gel as well as a shampoo, conditioner or personal wash composition, including a liquid or solid wash composition. In an especially preferred embodiment, the composition of this invention is a leave-on composition.

The active or component (including a triglyceride) that can yield, *in situ,* a product of oxidation means a polyunsaturated ingredient that oxidizes to yield volatile compounds, such as low molecular weight (<C10) aldehydes and/or ketones, especially hexanal. Remain chemically stable means at least 90% by weight of the component (based on total weight of the original total amount of component provided and as determined by HPLC using ASTM standards) remains intact and unoxidized in the composition for at least four weeks, and preferably, up to 8 weeks and less than 0.2 ppm, and preferably, less than 0.15 ppm hexanal is detected after composition with the component is stored at 45°C in an open jar for such respective amount of time. In a most preferred embodiment, the composition remains free of detectable aldehyde aroma for up to 8 weeks after being stored at 45°C and 3 months at room temperature in an open jar (as determined by a skilled panelist). Remains chemically stable, as used herein, is synonymous with free of malodor and color change in that an offensive odor is not detected when product is used by a consumer and a color change is not detected upon visual inspection under such conditions. Radical scavenger means an agent suitable to neutralize free radical oxidation products. Peroxide decomposer, as defined herein, means an agent that breaks down lipid peroxides that result from fatty acid oxidation. Oil-based composition means a composition that has an oil continuous phase, oil and is not an emulsion. Such a composition has less than 5% by weight water (and preferably, 0.0001 to 3% by weight water, and most preferably, no water). Oil carrier means an oil other than the component that can produce, *in situ,* a product of oxidation. The oil carrier is typically saturated.

Comprising as used herein is meant to include consisting essentially of and consisting of. Therefore, it is within the scope of this invention for the composition to consist essentially of or consist of a component that can produce, *in situ,* a product of oxidation, oil, radical scavenger and peroxide scavenger. Emulsion, as used herein, includes water-in-oil, oil-in-water, or double and triple emulsions. Oil-in-water emulsions are typically preferred and high internal phase emulsions are an option as described in commonly owned U.S. Patent Application Publication No. 2008/0311058. All ranges identified herein are meant to include all ranges subsumed therein if reference to the same is not explicitly made.

Except in the Examples or where otherwise explicitly indicated, all numbers used herein, including those indicating amounts or ratios of materials, are to be understood as modified by the word "about". For the avoidance of doubt, weight percent of a component or ingredient in a composition is meant to be based on the weight percent of the final composition desired whereby the same can be oil-based or an emulsion.

### Detailed Description of the Preferred Embodiments

Illustrative examples of the type of components (preferably active ingredients) that can produce, *in situ,* a product of oxidation and that may be used in this invention include polyunsaturated fatty acids like linoleic, and conjugated linoleic acid (CLA). Sunflower seed oil is also suitable for use wherein low oleic acid (i.e., ≤ 50%, and preferably, less than 40%, and most preferably, from 1o to 35% oleic acid based) sunflower seed oil is most desirable. CLA can comprise a group of positional and geometric isomers of linoleic acid in which various configurations of cis and trans double bonds at positions (6,8), (7,9), (8,10), (9,11), (10,12), (11,13), or mixtures thereof are possible. Therefore, many individual isomers and combinations of isomers may be used as the active ingredient (that yields malodor) in this invention.

A preferred CLA suitable for use in the compositions made in accordance with the present invention is the cis 9, trans 11 (hereinafter referred to as c9, t11) isomer. This particular isomer of the free acid has the structure shown below:

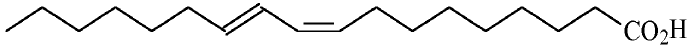

The invention also includes for use as actives derivatives of the free acid (which often comprise conjugated linoleic acid moieties) that can oxidize to generate a compound with malodor. Preferable derivatives include those derived from substitution of the carboxyl group of the acid, such as esters (e.g. triglyceride esters, monoglycerides esters, diglyceride esters), amides (e.g., ceramide derivatives), salts (e.g., alkali metal and alkali earth metal salts, ammonium salts); and/or those derived from substitution in the C18 carbon chain, such as alpha and/or beta alkoxy and/or hydroxy derivatives and the like .

In the case of triglyceride ester derivatives, all positional isomers of CLA substituents on the glycerol backbone are included. The triglycerides should contain at least one CLA moiety. For example, of the three esterifiable positions on the glycerol backbone, the 1 and 2 positions may be esterified with CLA and by another lipid at position 3 or as an alternative, the glycerol backbone could be esterified by CLA at the 1 and 3 positions with another lipid at position 2.

Wherever the term "conjugated linoleic acid" or "CLA" is used in this specification it is to be understood that the derivatives thereof comprising CLA moieties are also included. "CLA moieties" refer to CLA fatty acyl portion(s) of a CLA derivative.

By "c9, t11 isomer enriched CLA" is meant that at least about 30% by weight of the total CLA and/or CLA moieties present in the composition is in the form of the cis 9, trans 11 isomer. Preferably, at least about 35%, and most preferably, at least 40% to about 90% by weight of the total CLA and/or CLA moieties present in the composition, is in the form of the c9, t11 isomer, including all ranges subsumed therein.

However, in one particular preferred embodiment, cis 9, trans 11 isomer and trans 10, cis 12 isomer (or any derivatives thereof) are present as the active at a weight ratio from about 40:60 to about 60:40, and preferably, at a weight ratio from about 45:55 to about 55:45, including all ratios subsumed therein. CLA type products suitable for use in this invention are made available from suppliers like Stepan under the names Neobee® and Clarinol® as well as suppliers like BASF.

The CLA and/or derivatives thereof comprising CLA moieties according to the present invention may be prepared, for example, according to the method disclosed in WO 97/18320, the disclosure of which is incorporated herein by reference.

Typically, the amount of component that can produce, *in situ,* a product of oxidation that may be used in the oil-based composition of this invention is from 0.01 to 20%, and preferably, from 0.1 to 10%, and most preferably, from 1 to 4% by weight, based on total weight of the composition and including all ranges subsumed therein. In another desired embodiment, from 0.01 to 7.0%, and preferably, from 0.02 to 5%, and most preferably, from 0.5 to 3.5% by weight component that can produce, *in situ,* a product of oxidation is used based on total weight of the composition when the same is an emulsion. Most desirably, CLA, high linoleic sunflower seed oil or both, is/are the components that produce, *in situ,* the product of oxidation. When CLA is used, it is desirable for the composition of this invention to have less than 0.8% (preferably less than 0.55%) by weight in total of caprylic/capric triglyceride (CCT), isopropyl myristate and isopropyl palmitate. Preferably, no caprylic/capric triglyceride, isopropyl myristate and isopropyl palmitate is used when CLA is used. When both CLA and sunflower seed oil are used, such components are present at a weight ratio from 2:8 to 8:2, and preferably, at a weight ratio from 3:7 to 7:3, and most preferably, at a weight ratio from 4:6 to 6:4.

With respect to further radical scavenger that may be used in this invention, the same is limited only to the extent that it is suitable for use to stabilize a topical composition as defined herein and can be used in the presence of a peroxide decomposer. Illustrative examples include dibutylhydroxytoluene, rosemary extract, a tocopherol, a tocotrienol, marigold extract, octadecyl 3-(2,5-di-tert-butyl-4-hydroxyphenyl) propionate or a mixture thereof. The typically desired Vitamin E derivatives are alpha, beta, gamma and delta tocopherols and tocotrienols or mixtures thereof. Additional Vitamin E derivatives include tocopherylacetate, tocopherylacylate, -laurate, -myristate or - palmitate.

Another radical scavenger suitable for use includes, butylhydroxyanisole.

The amount of radical scavenger suitable for use in the composition of this invention is from 0.01 to 1.0%, and preferably, 0.05 to 0.7%, and most preferably, from 0.05 to 0.6%, based on total weight of the composition and including all ranges subsumed therein. In an often desired embodiment, from 0.05 to 0.2% by weight radical scavanger is used, based on total weight of the composition and including all ranges subsumed therein.

The peroxide decomposer that may further be used in this invention is limited only to the extent that it is suitable for use to stabilize a topical composition as defined herein with a radical scavenger. Illustrative examples include triphenyl phosphite, trioctadecylphosphite, diphenylalkyl phosphite, mixtures thereof or the like. The peroxide decomposer makes up from 0.01 to 1.0%, and preferably from 0.15 to 0.7%, and most preferably from 0.02 to 0.5% by weight of the composition. Especially preferred is when from 0.03 to 0.07 % by weight of the peroxide decomposer is used, based on total weight of the composition.

The radical scavenger employed in this invention is pentaerythritol tetrakis (3-(3,5-di-tert-butyl-4-hydroxyphenyl)propionate), and the peroxide decomposer is didodecyl 3,3' thiodipropionate. In a desired embodiment, the radical scavenger to peroxide decomposer weight ratio if from 2:8 to 8:2, and preferably, from 3:7 to 7:3, and most preferably, from 6:4 to 4:6. Radical scavengers are made commercially available by BASF under the names Tinogard® TT (= pentaerythritol tetrakis(3-(3,5-di-tert-butyl-4-hydroxyphenyl)propionate) and Tinogard® TS (= octadecyl 3-(2,5-di-tert-butyl-4-hydroxyphenyl)propionate), respectively. The peroxide decomposer didodecyl 3,3'thiodipropionate is made commercially available under the Tinogard® DA name, also by BASF. Most preferred radical scavenger and peroxide decomposer mixtures include rosemary extract and Tinogard® DA; Tinogard® TT and/or Tinogard® TS with Tinogard® DA, and dibutylhydroxytoluene and Tinogard® DA. Both radical scavenger and peroxide decomposer are used when the component that can produce *in situ* a product of oxidation is present in the composition at 3.5% by weight or more. It is, nevertheless, within the scope of the invention to optionally use radical scavenger and peroxide decomposer in compositions when the component that can produce *in situ* a product of oxidation is present at less than 3.5% by weight.

Cosmetically acceptable carriers suitable for use include water when the composition desired is not oil-based. Water is the most preferred additional carrier when the end use composition is an emulsion. Amounts of water may range from less than 5% to about 99%, and preferably, from about 5 to about 90%, and most preferably, from about 35 to about 80%, and optimally, from about 40 to about 75% by weight, based on total weight of the composition and including all ranges subsumed therein. Oil-in-water emulsions, again, are especially preferred

Other cosmetically acceptable carriers suitable for use in this invention may include mineral oils, silicone oils, synthetic or natural esters, and alcohols. Amounts of these materials may range from about 0.1 to about 50%, and preferably, from about 0.1 to about 30%, and most preferably, from about 1 to about 20% by weight of the composition, including all ranges subsumed therein.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from about 3 to about 9, and preferably, from about 4 to about 5 silicon atoms.

Linear volatile silicone materials generally have viscosities of less than about 5 centistokes at 25°C while cyclic materials typically have viscosities of less than about 10 centistokes.

Nonvolatile silicone oils useful as carrier material include polyalkyl siloxanes, polyalkylaryl siloxanes and polyether siloxane copolymers. The essentially nonvolatile polyalkyl siloxanes useful herein include, for example, polydimethylsiloxanes (like dimethicone-) with viscosities of from about 5 to about 100,000 centistokes at 25°C.

An often preferred silicone source is a cyclopentasiloxane and dimethiconol solution.

Among suitable esters are:
(1) Alkenyl or alkyl esters of fatty acids having 10 to 20 carbon atoms like octyl hydroxystearate, isopropyl isostearate, isononyl isonanonoate, oleyl myristate, oleyl stearate, and oleyl oleate;
(2) Ether esters- such as fatty acid esters of ethoxylated fatty alcohols;
(3) Polyhydroxy alcohol esters such as ethylene glycol mono- and difatty- acid esters, diethylene glycol mono and -difatty- acid esters, polyethylene glycol (200-6000) mono and -difatty- acid esters, propylene glycol mono and -difatty- acid esters, polypropylene glycol 2000 monooleate, polypropylene glycol 2000 monostearate, ethoxylated propylene glycol monostearate, glyceryl mono and difatty- acid esters, polyglycerol -polyfatty- esters, ethoxylated glyceryl monostearate, 1,3butylene glycol monostearate, 1,3butylene glycol distearate, polyoxyethylene polyol fatty acid ester, sorbitan- fatty acid esters, and polyoxyethylene -sorbitan fatty acid esters;
(4) Wax esters such as beeswax, spermaceti, myristyl myristate, stearyl stearate; and
(5) Sterol esters, of which soya sterol and cholesterol fatty acid esters are examples thereof.

Optional oils such as seabuckthorn oil, safflower oil, pistachio oil, and the like may also be used. These noted oils typically made up from 0.001 to 5% by weight of the composition where they are used, based on total weight of the composition and including all ranges subsumed therein.

Emulsifiers may be present in the emulsion compositions of the present invention. Total concentration of the emulsifier may range from 0.1 to 40%, and preferably, from 1 to 20%, and most preferably, from 1 to 5% by weight of the composition, including all ranges subsumed therein. The emulsifier may be selected from the group consisting of anionic, nonionic, cationic and amphoteric actives. Particularly preferred nonionic actives are those with a C₁₀-C₂₀ fatty alcohol or acid condensed with from about 2 to about 100 moles of ethylene oxide or propylene oxide; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids and amides; and polyoxyethylene sorbitan as well as combinations thereof. Alkyl polyglycosides and saccharide fatty amides (e.g. methyl gluconamides) are also suitable nonionic emulsifiers.

Preferred anionic emulsifiers include alkyl ether sulfate and sulfonates, alkyl sulfates and sulfonates, alkylbenzene sulfonates, alkyl and dialkyl sulfosuccinates, C₈-C₂₀ alkyl isothionates, C₈-C₂₀ alkoxy phosphates, alkylcarboxylates and combinations thereof.

Cationic emulsifiers that may be used include, for example, palmitamidopropyltrimonium chloride, distearyldimonium chloride and mixtures thereof. Useful amphoteric emulsifiers include cocoamidopropyl betaine, C₁₂-C₂₀ trialkyl betaines, sodium lauroamphoacetate, and sodium laurodiamphoacetate or a mixture thereof.

Other generally preferred emulsifiers include glyceryl stearate, glycol stearate, stearamide AMP, PEG-100 stearate, cetyl alcohol as well as emulsifying/thickening additives like hydroxyethylacrylate/sodium acryloyldimethyl taurates copolymer/squalane and mixtures thereof.

Preservatives can desirably be incorporated into the compositions of this invention to protect against the growth of potentially harmful microorganisms. Suitable traditional preservatives for compositions of this invention are alkyl esters of - parahydroxybenzoic- acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are iodopropynyl butyl carbamate, phenoxyethanol 1,2-alkane diols (like 1-2-octanediol), methyl paraben, ethyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from about 0.01% to about 2% by weight of the composition, including all ranges subsumed therein. In a desired embodiment, a preservative mixture of phenoxyethanol and 1,2-octanediol is used, typically at a weight ratio of 6:4 to 4:6.

Thickening agents may optionally be included in compositions of the present invention. Particularly useful are the polysaccharides. Examples include starches, natural/synthetic gums and cellulosics. Representative of the starches are chemically modified starches such as sodium hydroxypropyl starch phosphate and aluminum starch octenylsuccinate. Tapioca starch is often preferred. Suitable gums include xanthan, sclerotium, pectin, karaya, arabic, agar, guar, carrageenan, alginate and combinations thereof. Suitable cellulosics include hydroxypropyl cellulose, hydroxypropyl methylcellulose, ethylcellulose and sodium carboxy methylcellulose. Synthetic polymers are yet another class of effective thickening agent. This category includes crosslinked polyacrylates such as the Carbomers (Acrylates/ C₁₀₋₃₀ alkyl acrylate crosspolymer), polyacrylamides such as Sepigel® 305 and taurate copolymers such as Simulgel EG® and Aristoflex® AVC, the copolymers being identified by respective INCI nomenclature as Sodium Acrylate/Sodium Acryloyldimethyl Taurate and Acryloyl Dimethyltaurate/Vinyl Pyrrolidone Copolymer. Another preferred synthetic polymer suitable for thickening is an acrylate-based polymer made commercially available by Seppic and sold under the name Simulgel INS100.

Amounts of the thickener, when used, may range from about 0.001 to 5%, and preferably, from 0.1 to 2%, and most preferably, from about 0.2 to 0.5% by weight of the composition including all ranges subsumed therein.

Fragrances may optionally be included in compositions of the present invention. Each of these substances may range from about 0.05 to about 5%, preferably between 0.1 and 3% by weight.

Conventional humectants may be employed in the present invention. These are generally polyhydric alcohol-type materials. Typical polyhydric alcohols include glycerol (i.e., glycerine or glycerin), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. Most preferred is glycerin, propylene glycol or a mixture thereof. The amount of humectant employed may range anywhere from 0.5 to 20%, preferably between 1 and 15% by weight of the composition.

Monoenoic fatty acid (i.e., monounsaturated fatty acid) may optionally be employed with the component that can produce, *in situ,* a product of oxidation. Illustrative examples include cis-4-decenoic, cis-9-decenoic, cis-5-lauroleic, cis-4-dodecenoic, cis-9-tetradecenoic, cis-5-teradecenoic, cis-4-tetradecenoic, cis-9-hexadecenoic, cis-6-octadecenoic, cis-9-octadecenoic, tr-9-octadecenoic, cis-11-octadecenoic, cis-9-eicosenoic, cis-11-eicosenoic, cis-11-docosenoic, cis-13-docosenoic, cis-15-tetracosenoic acid, derivatives thereof or mixtures thereof.

The preferred optional monoenoic fatty acids (or salts or esters thereof) suitable for use in this invention are cis-6-octadecenoic acid (i.e., petroselinic acid) cis- and/or tri-9-octadecenoic acid (oleic) whereby the same may be used alone, in combination with other monoenoic fatty acids and/or in combination with CLA and/or in combination with other active components defined herein, including sunflower seed oil. In another preferred embodiment, an ester of cis- and/or tri-9-octadecenoic acid is used, and especially, a triglyceride thereof.

If used, the amount of monoenoic acid used in the invention is typically 70% less, and preferably, 60% less, and most preferably, 50% less than the amount of polyunsaturated fatty acid (or ester or salt thereof) used, based on total weight of oil-based compositions and emulsions of this invention.

Compositions of the present invention may include skin benefit vitamins. Illustrative vitamins included Vitamin B₂, Vitamin B₃ (niacinamide), Vitamin B₆, Vitamin D and K. Derivatives of the vitamins may also be employed. Total amount of vitamins when present in compositions according to the present invention may range from 0.001 to 10%, preferably from 0.01% to 1%, optimally from 0.1 to 0.5% by weight of the composition, and most preferably from 0.01 to 3% by weight of the composition.

Another optional additive suitable for use in this invention includes creatine and its derivatives, 12-hydroxysteatic acid, mixtures thereof or the like. Such additives, when used, collectively make up from about 0.001 to about 5% by weight of the composition.

Desquamation promoters may be present. Illustrative are the alpha-hydroxycarboxylic acids, beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic and its derivatives, lactic and malic acids. Salicylic acid is representative of the beta-hydroxycarboxylic acids. Amounts of these materials when present may range from 0.01 to 4% by weight of the composition.

A variety of herbal extracts may optionally be included in compositions of this invention. The extracts may either be water soluble or water-insoluble carried in a solvent which respectively is hydrophilic or hydrophobic. Water and ethanol are the preferred extract solvents. Illustrative extracts include those from yarrow, chamomile, licorice, aloe vera, grape seed, citrus unshui, willow bark, sage and thyme.

Also optionally suitable for use include materials like chelators (e.g., EDTA), opacifiers (like TiO₂, particle size from 50 to 1200 nm, and preferably, 50 to 350 nm), C₈₋₂₂ fatty acid substituted saccharides, lipoic acid, retinoxytrimethylsilane (available from Clariant Corp. under the Silcare 1M-75 trademark), dehydroepiandrosterone (DHEA) and combinations thereof. Amounts of these materials, when used, may range from 0.001 to 10%, preferably from 0.001 to 1% by weight of the composition.

Sunscreen actives may also be included in compositions of the present invention. Particularly preferred are such materials as octocrylene, ethylhexyl p-methoxycinnamate, available as Parsol MCX®, Avobenzene, available as Parsol 17890, benzophenone-3, also known as Oxybenzone, octyl salicylate, and homosalate. Inorganic sunscreen actives may be employed such as microfine titanium dioxide, zinc oxide, polyethylene and various other polymers. Amounts of the sunscreen agents when present may generally range from 0.1 to 30%, preferably from 0.5 to 20%, optimally from 0.75 to 10% by weight.

Conventional buffers/pH modifiers may be used. These include commonly employed additives like sodium hydroxide, potassium hydroxide, hydrochloric acid, citric acid and citrate/citric acid buffers. In an especially preferred embodiment, the pH of the composition of this invention is from 4 to 8, and preferably, from 4.25 to 7.75, and most preferably, from 6 to 7.5, including all ranges subsumed therein.

The compositions of the present invention, when topically applied, are suitable to improve a variety of skin characteristics. This is particularly true since the components that can produce, *in situ,* products of oxidation are typically skin benefit actives. These actives can moisturize, reduce wrinkles, contribute to even skin tone and overall improve the elasticity of skin.

A wide variety of packaging can be employed to store and deliver the composition of this invention. Packaging is often dependent upon the type of personal care end-use. For instance, leave-on skin lotions and creams, shampoos, conditioners and shower gels can surprisingly be packaged in plastic containers with a cap/cover or an opening at a dispensing end covered by a closure. Suitable closures are screw-caps, non-aerosol pumps and flip-top hinged lids. Packaging for antiperspirants, deodorants and depilatories may involve a container with a roll-on ball on a dispensing end. Alternatively these types of personal care products may be delivered in a stick composition formulation in a container with propel-repel mechanism where the stick moves on a platform towards a dispensing orifice. Metallic cans pressurized by a propellant and having a spray nozzle serve as packaging for antiperspirants, shave creams and other personal care products. Toilette bars may have packaging constituted by a cellulosic or plastic wrapper or within a cardboard box or even encompassed by a shrink wrap plastic film. Surprisingly, the compositions of the present invention do not require airless packaging.

The following examples are provided to facilitate an understanding of the present invention. The examples are not intended to limit the scope of the claims.

### Example 1

Components capable of producing, *in* situ, a product of oxidation, radical scavengers and peroxide decomposers were added to a lotion base as described below and to produce each Sample. The compositions of each Sample were prepared by mixing under conditions of moderate shear at atmospheric pressure and room temperature.

**BASE**

| Ingredient Name | % of Formula |
|---|---|
| Water | Balance |
| Disodium EDTA | 0.05 |
| Preservative | 0.7 |
| Glycerin | 10.0 |
| Thickener | 0.18 |
| Sodium Hydroxide | 0.24 |
| Stearic Acid | 0.63 |
| Glycol Stearate (and) Stearamide AMP | 0.87 |
| Cetyl Alcohol | 0.48 |
| Glyceryl Stearate | 0.84 |
| PEG-100 Stearate | 0.74 |
| Mineral Oil | 3.0 |
| Vitamin B3 | 3.0 |
| Component that can produce, *in situ,* a product of oxidation | As indicated in the Tables below |
| Radical Scavenger* | As indicated in the Tables below |
| Peroxide decomposer* | As indicated in the Tables below |
| Fragrance | 0.15 |

| | |
|---|---|
| *solubilized in oil prior to adding | |

The samples were stored at 45°C and greater than 90% of the active originally provided remained intact and unoxidized for eight (8) weeks (as determined by HPLC, using ASTM standards).
Where stabilization has been confirmed, the compositions were free of detectable malodor and visual color change, and had characteristic sensory attributes when applied to trained panelists. The Samples were also free of malodor after storage for three months at room temperature.

**TABLE I**

| **CLA - Component that can, *in situ,* produce a product of oxidation at 3 weight percent** | | | | | |
|---|---|---|---|---|---|
| **Sample** | **Radical Scavenger and Peroxide Decomposer** | **(wt %)** | **Stabilization** | **Hexanal Concentration (PPM)**** | **CLA recovery, (%)***** |
| 1 | BHT | 0.1 | Yes | 0.04 | 96.5 |
| 2 | Alpha-tocopherols | 0.1 | Yes | 0.08 | 93.6 |
| 3 | Vitamin C | 0.1 | No | fail | n/a |
| 4 | Rosemary Extract | 0.1 | Yes | 0.06 | 97.6 |
| 5 | Green Tea Extract | 0.1 | No | fail | 98.5 |
| 6 | Tinogard TT | 0.1 | Yes | 0.04 | 96.4 |
| 7 | Tinogard DA | 0.1 | Yes | 0.05 | 95.2 |
| 8 | BHT | 0.05 | Yes | 0.04 | 98.1 |
| | Tinogard DA | 0.05 | | | |
| 9 | Tocopherols | 0.05 | Yes | 0.03 | 97.3 |
| | Tinogard DA | 0.05 | | | |
| 10 | Vitamin C | 0.05 | No | fail | n/a |
| | Tinogard DA | 0.05 | | | |
| 11 | Rosemary Extract | 0.05 | Yes | 0.05 | 95.0 |
| | Tinogard DA | 0.05 | | | |
| 12 | Green Tea | 0.05 | No | fail | 97.5 |
| | Tinogard DA | 0.05 | | | |
| 13 | Tinogard TT | 0.05 | Yes | 0.03 | 97.3 |
| | Tinogard DA | 0.05 | | | |
| 14 | Tinogard TT | 0.05 | No* | 0.4 | 98.7 |
| | Tinogard DA | 0.05 | | | |
| 15 | Tinogard TT | 0.05 | Yes** | 0.02 | 97.6 |
| | Tinogard DA | 0.05 | | | |

| | | | | | |
|---|---|---|---|---|---|
| fail=formula separation * formulation made with 3% CCT in lieu of mineral oil (CCT yellowed and apparently contaminated) ** formulation made with 3% CCT in lieu of mineral oil (CCT transparent, not contaminated) *** 0-0.2ppm - nonperceivable odor, 0.2-0.3 - trace detection of odor, >0.3ppm - malodor detected **** CLA raw material (as supplied) used as a standard for HPLC analysis | | | | | |

**TABLE II**

| **Low Oleic <40% Oleic Sunflower Seed Oil Active at 3 weight percent.** | | | | |
|---|---|---|---|---|
| **Sample** | **Radical Scavenger and Peroxide Decomposer** | **(wt %)** | **Stabilization** | **Hexanal Concentration (PPM)** |
| 1 | BHT | 0.1 | Yes | 0.13 |
| 2 | Alpha-tocopherols | 0.1 | No | 0.23 |
| 3 | Vitamin C | 0.1 | No | fail |
| 4 | Rosemary Extract | 0.1 | No | 0.26 |
| 5 | Green Tea | 0.1 | No | fail |
| 6 | Tinogard TT | 0.1 | No | 0.22 |
| 7 | Tinogard DA | 0.1 | Yes | 0.06 |
| 8 | BHT | 0.05 | Yes | 0.06 |
| | Tinogard DA | 0.05 | | |
| 9 | Tocopherols | 0.05 | Yes | 0.1 |
| | Tinogard DA | 0.05 | | |
| 10 | Vitamin C | 0.05 | No | fail |
| | Tinogard DA | 0.05 | | |
| 11 | Rosemary Extract | 0.05 | Yes | 0.15 |
| | Tinogard DA | 0.05 | | |
| 12 | Green Tea | 0.05 | No | fail |
| | Tinogard DA | 0.05 | | |
| 13 | Tinogard TT | 0.05 | Yes | 0.01 |
| | Tinogard DA | 0.05 | | |
| 14 | Tinogard TT | 0.05 | No* | >0.3 |
| | Tinogard DA | 0.05 | | |
| 15 | Tinogard TT | 0.05 | Yes** | 0.04 |
| | Tinogard DA | 0.05 | | |

| | | | | |
|---|---|---|---|---|
| fail = formula separation *formulation made 3% CCT in lieu of mineral oil (CCT yellowed and apparently contaminated) ** formulation made with 3% CCT in lieu of mineral oil (CCT transparent, not contaminated) | | | | |

**TABLE III**

| **Low Oleic (≤ 40% oleic) Sunflower Seed Oil and CLA Active at 3 weight percent each.** | | | | | |
|---|---|---|---|---|---|
| **Sample** | **Radical Scavenger and Peroxide Decomposer** | **(wt %)** | **Stabilization** | **Hexanal Concentration (PPM)*** | **CLA recovery, (%)**** |
| 1 | BHT | 0.1 | No | 0.04 | 94.3 |
| 2 | Alpha-tocopherols | 0.1 | No | 0.8 | 91.6 |
| 3 | Vitamin C | 0.1 | No | fail | n/a |
| 4 | Rosemary Extract | 0.1 | No | 0.4 | 93.7 |
| 5 | Green Tea | 0.1 | No | fail | n/a |
| 6 | Tinogard TT | 0.1 | No | 0.2 | 94.9 |
| 7 | Tinogard DA | 0.1 | No | 0.21 | 90.5 |
| 8 | BHT | 0.05 | Yes | 0.08 | 97.6 |
| | Tinogard DA | 0.05 | | | |
| 9 | Tocopherols | 0.05 | Yes | 0.07 | 92.5 |
| | Tinogard DA | 0.05 | | | |
| 10 | Vitamin C | 0.05 | No | fail | n/a |
| | Tinogard DA | 0.05 | | | |
| 11 | Rosemary Extract | 0.05 | Yes | 0.11 | 95.8 |
| | Tinogard DA | 0.05 | | | |
| 12 | Tinogard TT | 0.05 | Yes | 0.09 | 96.9 |
| | Tinogard DA | 0.05 | | | |

| | | | | | |
|---|---|---|---|---|---|
| fail=formula separation * 0-0.2ppm - nonperceivable odor, 0.2-0.3 - trace detection of odor, >0.3ppm - malodor detected ** CLA raw material (as supplied) used as a standard for HPLC analysis | | | | | |

The data in the Tables above again confirms the unexpected and surprising results obtained in this invention where compositions having particular radical scavengers and peroxide decomposers stabilized components that are suitable to generate, *in situ,* products of oxidation. As determined by trained panelists, the stabilized compositions made consistent with this invention were free of malodor subsequent to inspection and did not lose their conventional sensory characteristics after topical application. The compositions were also free from discoloration at the given conditions as determined by visual inspection.

## Claims

1. A topical composition comprising:
(a) a component that can produce *in situ* a product of oxidation, the component comprising polyunsaturated fatty acid, ester or salt thereof, the component further comprising conjugated linoleic acid, low oleic sunflower seed oil or both;
(b) a 0.1 to 1 weight percent radical scavenger comprising pentaerythritol tetrakis-(3-(3,5-di-tert-butyl-4-hydroxyphenyl)propionate) and 0.1 to 1 weight percent peroxide decomposer comprising didodecyl 3,3' thiodipropionate; and
(c) oil carrier comprising caprylic/capric triglyceride.

2. The composition according to claim 1 wherein the polyunsaturated component is conjugated linoleic acid.

3. The composition according to claim 1 wherein the composition is oil-based and not an emulsion.

4. The composition according to claim 1 wherein the composition represents an oil phase in an emulsion.

5. The composition according to claim 1 wherein the radical scavenger further comprises rosemary extract, a tocopherol, a tocotrienol, marigold extract, octadecyl 3-(2,5-di-tert-butyl-4-hydroxyphenyl) propionate or a mixture thereof.

6. The composition according to claim 1 wherein the radical scavenger further comprises butyl hydroxyanisole.

7. The composition according to claim 1 wherein the peroxide decomposer further comprises, triphenyl phosphite, trioctadecylphosphite, diphenyl alkyl phosphite or a mixture thereof.

8. The composition according to claim 1 wherein the polyunsaturated component is conjugated linoleic acid, the radical scavenger is pentaerythritol tetrakis-(3-(3,5-di-tert-butyl-4-hydroxyphenyl)propionate and the peroxide decomposer is didodecyl 3,3'thiodipropionate.

9. The composition according to claim 1 wherein the radical scavenger makes up from 0.05 to 0.7% by weight of the composition, the peroxide decomposer makes up from 0.15 to 0.7% by weight of the composition and the component that can produce, *in situ,* a product of oxidation makes up from 0.1 to 20% by weight of the composition when the composition is oil-based and 0.01 to 7% when the composition is an emulsion.

10. The composition according to claim 9 wherein the composition comprises less than 0.8% by weight in total of caprylic/capric triglyceride, isopropylmyristate and isopropyl palmitate when the component that can produce, *in situ,* a product of oxidation comprises conjugated linoleic acid.

11. The composition according to claim 1 wherein the composition further comprises niacinamide, petroselinic acid or both.

12. A non-therapeutic method for cosmetically treating skin comprising the step of contacting skin with the composition of claim 1.

13. Use of the composition of claim 9 to moisturize skin, reduce skin wrinkles, and/or to improve even skin tone.

14. The composition according to claim 1 wherein the radical scavenger further comprises dibutylhydroxytoluene.

## Patentansprüche

1. Topische Zusammensetzung, umfassend:
(a) eine Komponente, die *in situ ein* Oxidationsprodukt erzeugen kann, wobei die Komponente mehrfach ungesättigte Fettsäure, Ester oder Salz davon umfasst, wobei die Komponente ferner konjugierte Linolsäure, Sonnenblumenkernöl mit niedrigem Ölsäure-Gehalt oder beide umfasst;
(b) 0,1 bis 1 Gewichtsprozent Radikalfänger, umfassend Pentaerythrit-tetrakis-(3-(3,5-di-tert-butyl-4-hydroxyphenyl)propionat) und 0,1 bis 1 Gewichtsprozent Peroxidzersetzer, umfassend Didodecyl-3,3'-thiodipropionat und
(c) einen Ölträger, umfassend Capryl/Caprin-Triglycerid.

2. Zusammensetzung nach Anspruch 1, wobei die mehrfach ungesättigte Komponente konjugierte Linolsäure ist.

3. Zusammensetzung nach Anspruch 1, wobei die Zusammensetzung auf Öl beruht und keine Emulsion ist.

4. Zusammensetzung nach Anspruch 1, wobei die Zusammensetzung eine Ölphase in einer Emulsion darstellt.

5. Zusammensetzung nach Anspruch 1, wobei der Radikalfänger ferner Rosmarinextrakt, Tocopherol, Tocotrienol, Ringelblumenextrakt, Octadecyl-3-(2,5-di-tert-butyl-4-hydroxyphenyl)propionat oder eine Mischung davon umfasst.

6. Zusammensetzung nach Anspruch 1, wobei der Radikalfänger ferner Butylhydroxyanisol umfasst.

7. Zusammensetzung nach Anspruch 1, wobei der Peroxidzersetzer ferner Triphenylphosphit, Trioctadecylphosphit, Diphenylalkylphosphit oder eine Mischung davon umfasst.

8. Zusammensetzung nach Anspruch 1, wobei die mehrfach ungesättigte Komponente konjugierte Linolsäure, der Radikalfänger Pentaerythrit-tetrakis-(3-(3,5-di-tert-butyl-4-hydroxyphenyl)propionat und der Peroxidzersetzer Didodecyl-3,3'-thiodipropionat ist.

9. Zusammensetzung nach Anspruch 1, wobei der Radikalfänger von 0,05 bis 0,7 Gewichts-% der Zuammensetzung ausmacht, der Peroxidzersetzer von 0,15 bis 0,7 Gew.-% der Zusammensetzung ausmacht und die Komponente, die *in situ ein* Oxidationsprodukt erzeugen kann, von 0,1 bis 20 Gewichts-% der Zusammensetzung ausmacht, wenn die Zusammensetzung auf Öl beruht, und 0,01 bis 7 Gewichts-%, wenn die Zusammensetzung eine Emulsion ist.

10. Zusammensetzung nach Anspruch 9, wobei die Zusammensetzung insgesamt weniger als 0,8 Gewichts-% Capryl/Caprin-Triglycerid, Isopropylmyristat und Isopropylpalmitat enthält, wenn die Komponente, die *in situ ein* Oxidationsprodukt bilden kann, konjugierte Linolsäure umfasst.

11. Zusammensetzung nach Anspruch 1, wobei die Zusammensetzung ferner Niacinamid, Petroselinsäure oder beides umfasst.

12. Nicht-therapeutisches Verfahren zur kosmetischen Hautbehandlung, umfassend den Schritt des Inkontaktbringens der Haut mit der Zusammensetzung des Anspruchs 1.

13. Verwendung der Zusammensetzung des Anspruchs 9 zum Befeuchten von Haut, zum Reduzieren von Hautfalten und/oder zum Verbessern des gleichmäßigen Hauttons.

14. Zusammensetzung nach Anspruch 1, wobei der Radikalfänger ferner Dibutylhydroxytoluol umfasst.

## Revendications

1. Composition topique comprenant :
(a) un constituant qui peut produire *in situ* un produit d'oxydation, le constituant comprenant un acide gras polyinsaturé, ester ou sel de celui-ci, le constituant comprenant de plus de l'acide linoléique conjugué, de l'huile de graines de tournesol à faible teneur oléique ou les deux ;
(b) de 0,1 à 1 pourcent en masse de fixateur de radical comprenant du tétrakis-(3-(3,5-di-tert-butyl-4-hydroxyphényl)propionate de pentaérythritol et de 0,1 à 1 pourcent en masse de désintégrateur de peroxyde comprenant du 3,3'-dithiodipropionate de didodécyle ; et
(c) un support d'huile comprenant un triglycéride caprylique/caprique.

2. Composition selon la revendication 1, dans laquelle le constituant polyinsaturé est l'acide linoléique conjugué.

3. Composition selon la revendication 1, dans laquelle la composition est à base d'huile et n'est pas une émulsion.

4. Composition selon la revendication 1, dans laquelle la composition représente une phase d'huile dans une émulsion.

5. Composition selon la revendication 1, dans laquelle le fixateur de radical comprend de plus de l'extrait de romarin, un tocophérol, un tocotriénol, de l'extrait de souci, du 3-(2,5-di-tert-butyl-4-hydroxyphényl)propionate d'octadécyle ou un mélange de ceux-ci.

6. Composition selon la revendication 1, dans laquelle le fixateur de radical comprend de plus du butylhydroxyanisole.

7. Composition selon la revendication 1, dans laquelle l'agent de décomposition de peroxyde comprend de plus du triphénylphosphite, trioctadécylphosphite, diphénylalkylphosphite ou un mélange de ceux-ci.

8. Composition selon la revendication 1, dans laquelle le constituant polyinsaturé est de l'acide linoléique conjugué, le fixateur de radical est le tétrakis-(3-(3,5-di-tert-butyl-4-hydroxyphényl)propionate de pentaérythritol et le désintégrateur de peroxyde est le 3,3'-thiopropionate de didodécyle.

9. Composition selon la revendication 1, dans laquelle le fixateur de radical constitue de 0,05 à 0,7 % en masse de la composition, le désintégrateur de peroxyde constitue de 0,15 à 0,7 % en masse de la composition et le constituant qui peut produire, *in situ,* un produit d'oxydation constitue de 0,1 à 20 % en masse de la composition lorsque la composition est à base d'huile et de 0,01 à 7 % lorsque la composition est une émulsion.

10. Composition selon la revendication 9, dans laquelle la composition comprend moins de 0,8 % en masse au total de triglycéride caprylique/caprique, myristate d'isopropyle et palmitate d'isopropyle lorsque le constituant qui peut produire, *in situ,* un produit d'oxydation comprend de l'acide linoléique conjugué.

11. Composition selon la revendication 1, dans laquelle la composition comprend de plus du niacinamide, de l'acide pétrosélinique ou les deux.

12. Procédé non-thérapeutique pour le traitement cosmétique de la peau comprenant l'étape de mise en contact de la peau avec la composition de la revendication 1.

13. Utilisation de la composition de la revendication 9 pour hydrater la peau, réduire les rides de la peau, et/ou améliorer l'uniformité du teint.

14. Composition selon la revendication 1, dans laquelle le fixateur de radical comprend de plus du dibutylhydroxytoluène.
